# EUROPEAN PATENT SPECIFICATION

(11) **EP 0 662 130 B1**
(45) Date of publication and mention of the grant of the patent: **26.11.1997**
(21) Application number: 93921471.4
(22) Date of filing: 15.09.1993
(51) Int. Cl.: C12N 15/12, C12N 15/85, C07K 14/00, A61K 38/00

(54) **A method of producing hepatocyte growth factor and a cell line**
Eine Methode zur Herstellung von Hepatozytwachstumsfaktor und eine Zelllinie
Méthode pour la préparation du facteur de croissance d'hépatocytes et une ligne céllulaire

(30) Priority: 18.09.1992 US 946061
(43) Date of publication of application: 12.07.1995
(60) Divisional application: 97201105.0
(73) Proprietor: THE GOVERNMENT OF THE UNITED STATES OF AMERICA as represented by THE SECRETARY OF THE DEPARTMENT OF HEALTH AND HUMAN SERVICES, Rockville, MD 20892-9902 (US)
(72) Inventor: FALETTO, Donna L., Mount Airy, MD 21771 (US); TSARFATY, Ilan, Frederick, MD 21702 (US); RONG, Sing, Frederick, MD 21702 (US); OSKARSSON, Marianne, Gaithersburg, MD 20879 (US); VANDE WOUDE, George F., Berryville, VA 22611 (US)
(74) Representative: Plougmann, Vingtoft & Partners A/S
(86) International application number: US9308531
(87) International publication number: WO9406909

(56) References cited:
- EP-A- 0 456 188
- WO-A-92/05184
- WO-A-92/13097
- WO-A-93/15754
- EMBO JOURNAL vol. 11, no. 7 , July 1992 , IRL PRESS LIM., OXFORD, ENGL.; pages 2503 - 2510 N.A. LOKKER ET AL. 'Structure-function analysis of hepatocyte growth factor: identification of variants that lack mitogenic activity yet retain high affinity receptor binding' cited in the application
- SCIENCE vol. 218 , 10 December 1982 , AAAS, WASHINGTON, DC, US; pages 1122 - 1125 D.G. BLAIR ET AL. 'New method for detecting cellular transforming genes' cited in the application
- BIOCHEM. BIOPHYS. RES. COMMUN. vol. 180, no. 2 , 31 October 1991 , ACADEMIC PRESS, N.Y., US; pages 1151 - 1158 N. SHIMA ET AL. 'Tumor cytotoxic factor/Hepatocyte growth factor from human fibroblasts: Cloning of its cDNA, purification and characterization of recombinant protein'
- MOL. CELL. BIOL. vol. 12, no. 11 , November 1992 , AM. SOC. MICROBIOL., WASHINGTON, D.C. US; pages 5152 - 5158 S. RONG ET AL. 'Tumorigenicity of the met proto-oncogene and the gene for hepatocyte growth factor'
- CELL GROWTH & DIFFERENTIATION vol. 4, no. 7 , July 1993 , WAVERLY PRESS, INC., BALTIMORE,MD,US; pages 563 - 569 S. RONG ET AL. 'Tumorigenesis induced by coexpression of human hepatocyte growth factor and the human met protooncogene leads to high levels of expression of the ligand and the receptor'

## Description

### Background of the Invention

Hepatocyte growth factor (HGF) was first purified from human and rabbit plasma and rat platelets on the basis of its ability to stimulate mitogenesis of rat hepatocytes. (E. Gohda *et al., J. Clin. Invest* 81: 414 (1988); R. Zarnegar and G. Michalopoulos, *Cancer Res.* 49: 3314 (1989); T. Nakamura *et al. FEBS Lett.* 224: 311 (1987)). Thus, HGF may act as a humoral factor promoting liver regeneration after partial hepatectomy or liver injury. (E.H. Gohda *et al., J. Clin. Invest.* 81: 414-419 (1988); G.K. Michalopoulos, *FASEB J.* 4: 176 (1990)). The same factor was purified from human fibroblast culture medium and shown to act on melanocytes and a variety of epithelial and endothelial cells. (T. Igawa *et al., BBRC* 174: 831-838 (1991); M. Kan et *al., BBRC* 174: 331-337 (1991) and J.S. Rubin *et al., Proc. Nat'l. Acad. Sci. U.S.A.* 88: 415 (1990)). Together with evidence of HGF expression in several organs (J.S. Rubin *et al., Proc. Nat'l. Acad. Sci. U.S.A.* 88: 415 (1990); K. Tashiro *et al. Proc. Nat'l. Acad. Sci. U.S.A.* 87: 3200 (1990); R. Zarnegar *et al., Proc. Nat'l. Acad. Sci. U.S.A.* 87: 1252 (1990); T. Kinoshita *et al., Biochem. Biophys. Res. Comm.* 165: 1229 (1989)), these findings indicate that HGF may also act as a paracrine mediator of proliferation for a broad spectrum of cell types. Molecular cloning of HGF revealed a remarkable structural homology to plasminogen and related serine proteases (J.S. Rubin *et al., Proc. Nat'l. Acad. Sci. U.S.A.* 88: 415 (1990); T. Nakamura *et al., Nature* 342: 440 (1989); K. Miyazawa et *al., Biophys. Res. Comm.* 163: 967 (1989)). Recent evidence that HGF induces rapid tyrosine phosphorylation of proteins in intact target cells suggests that a tyrosine kinase receptor mediates its mitogenic signal (J.S. Rubin *et al., Proc. Nat'l. Acad. Sci. U.S.A. 88:* 415 (1990)).

HGF is structurally related to the family of serine proteases that includes plasminogen, prothrombin, urokinase, and tissue plasminogen activator (J.S. Rubin *et al., Proc. Nat'l. Acad. Sci. U.S.A.* 88: 415 (1990); T. Nakamura *et al., Nature* 342: 440 (1989)). For instance, HGF structurally resembles plasminogen in that it possesses characteristic kringle domains (Patthy *et al. FEBS Lett.* 171: 131-136 (1984)) and a serine protease domain (Miyazawa *et al., Biochem. Biophys. Res. Commun.* 163: 967-73 (1989); Nakamura *et al., Nature* 342: 440-43 (1989)). As defined in the present invention, HGF includes a growth factor previously characterized as a broad-spectrum mitogen called plasminogen-like growth factor (PLGF), the subject matter of U.S. application serial no. 07/582,063. Several proteases, including members of the serine protease family, stimulate DNA synthesis presumably through a proteolytic mechanism similar to tryptic activation of the insulin receptor. (S.E. Shoelson *et al. J. Biol. Chem.* 263: 4852 (1988)). Only urokinase has been found to associate with a specific cell-surface receptor, which itself bears no homology to any known tyrosine kinase receptors (A.L. Roldan *et al., EMBO J.* 9: 467 (1990)).

Scatter factor (SF) originally had been considered to be related to but different from HGF, SF being associated with cell motogenicity (motility), and HGF being associated with cell mitogenicity (growth). However, recent work has demonstrated that SF and HGF are, in fact, the same protein having the identical amino acid sequence, the same receptor which is the protein encoded by the *c-met* proto-oncogene, and same biochemical affect (E. Gherardi and M. Stoker, *Nature* 346: 228 (1990); K.M. Weidner *et al., PNAS* 88: 7001-7005 (1991); M. Bhargava, *et al., Cell Growth & Diffn.* 3(1): 11-20 (1992); Naldini *et al., EMBO J.* 10(10): 2867-78 (1991); E. Gherardi and M. Stoke, *Cancer Cells* 3:(6): 227-32 (1991) and Graziani *et al., J. Biol. Chem. (USA)* 266

The subject matter of U.S. Serial No. 07/642,971, incorporated by reference above, describes a complex comprising HGF/SF and *met* protooncogene protein ("Met"), and identifies Met as the receptor for HGF/SF. The *met* protooncogene protein is a member of the tyrosine kinase growth factor receptor family. Knowledge of this receptor/ligand relationship facilitates the study of proliferative disorders and tumorigenicity in which expression of these molecules may play an important role. Additionally, identification of the *met* protooncogene receptor HGF/SF complex provides a means for identifying tissues other than liver tissue affected by factor binding.

Evidence suggests that the positive affects of growth factors on cell proliferation can be counteracted at a variety of levels, both intracellularly (Moses et *al. Cell* 63: 245-247 (1990)) and at the cell surface (Hannum *et al., Nature* 343: 336-340 (1990); Eisenberg, *et al.. Nature* 343: 341-346 (1990); Carter *et al., Nature* 344:633-637 (1990)).

Various sources of human HGF have been identified (Nakamura, T., *Progress in Growth Factor Research,* 3: 67-86 (1992)) and it has been shown that the gene product can be overexpressed when transfected into *Cos* cells or by baculovirus host systems. (Nakamura et *al., Nature* 342:440-43 (1989); Cooper, *et al., The EMBO J.,* 5(10): 2623-2628 (1986)). A mammalian cell line continuously shedding large amounts of human HGF/SF has yet to be identified.

### Summary of the Invention

In view of need for a continuously producing source of large quantities of HGF/SF, applicants have discovered and describe herein a mouse NIH/3T3 cell line which can express unexpectedly high levels of HGF/SF by recombinant methods. Thus, one embodiment of the invention relates to a method of producing HGF/SF comprising the steps of:
(a) transfecting NIH/3T3 cells with DNA encoding HGF/SF^{hu} and Met^{hu};
(b) introducing cells transfected in accordance with step (a) into a mammal, thereby generating a primary tumor;
(c) explanting and propagating cells of the primary tumor *in vitro;*
(d) selecting those cells propagated in accordance with step (c) which express high levels of HGF/SF^{hu} and high levels of Met^{hu};
(e) introducing cells selected in accordance with step (d) into a mammal, thereby producing a secondary tumor;
(f) explanting and propagating cells of the secondary tumor *in vitro;* and
(g) obtaining HGF/SF^{hu} produced by the cells of step (f).

Yet another embodiment of the invention relates to a cell line co-transfected with a first DNA vector comprising, in operable linkage, a promoter derived from a long terminal repeat of a retrovirus, DNA encoding the entire coding domain of human Met and a polyadenylation signal and a second DNA vector comprising, in operable linkage, a promoter derived from a long terminal repeat of a retrovirus, DNA encoding human HGF/SF and a polyadenylation signal.

### Brief Description of the Drawings

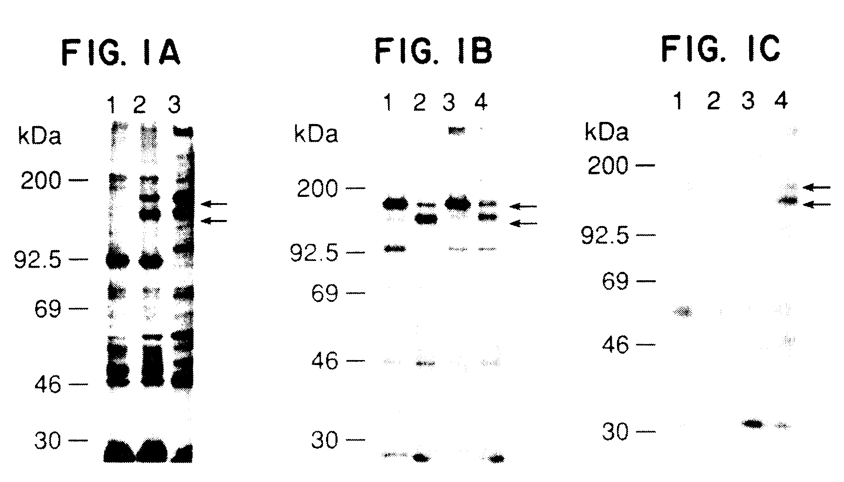

Figure 1 demonstrates *met* products in transfected NIH/3T3 cells. (A) **Immunoprecipitation analysis**. Cells were metabolically labeled with [³⁵S]methionine and [³⁵S]cysteine (Translabel, ICN) for 5 hours in DMEM lacking methionine and cysteine (Gibco) and cell lysates were immunoprecipitated with either a Met^{hu}-specific monoclonal antibody, 19S (Faletto, D.L. et *al., Oncogene* 7: 1149-1157 (1991) (lanes 1 and 2), or Met^{mu}-specific peptide antibody, SP260 (Iyer, A. *et al., Cell Growth & Diff. 1:* 87-95 (1990)) (lane 3). Immunoprecipitation was completed with protein G-sepharose (Gibco), the complex was solubilized in SDS sample buffer with 5% β-mercaptoethanol, and resolved on 7.5% acrylamide gels. In lane 1, cells were transfected with pSV2neo only; in lane 2, cells were transfected with *met*^{hu}; in lane 3, cells were transfected with *met*^{mu}*.* (B) **Pulse-chase analysis.** Cells were metabolically labeled with [³⁵S]methionine and [³⁵S]cysteine for 45 minutes (lanes 1 and 3), followed by a chase period of 3 hours (lanes 2 and 4). Met was immunoprecipitated with 19S monoclonal antibody against Met^{hu} (lanes 1 and 2) or SP260 peptide antibody, against Met^{mu} (lanes 3 and 4) and subjected to electrophoresis as in panel A. In lanes 1 and 2 are cells transfected with *Met*^{hu}*;* in lanes 3 and 4 are cells transfected with *Met*^{mu}*.* (C) **Cell surface iodination of met.** Near-confluent cells were pelleted and labeled with Na¹²⁵I in the presence of Iodo-Gen (Pierce). The labeled cells were washed three times with PBS, lysed with RIPA buffer, and subjected to immunoprecipitation with either 19S monoclonal antibody (lanes 1 and 2) or SP260 peptide antibody (lanes 3 and 4). Met^{hu} expressing NIH/3T3 cells are in lanes 1 and 3. Met^{mu} expressing NIH/3T3 cells are in lanes 2 and 4. Arrows indicate the positions of p170^{met} and p140^{met}.

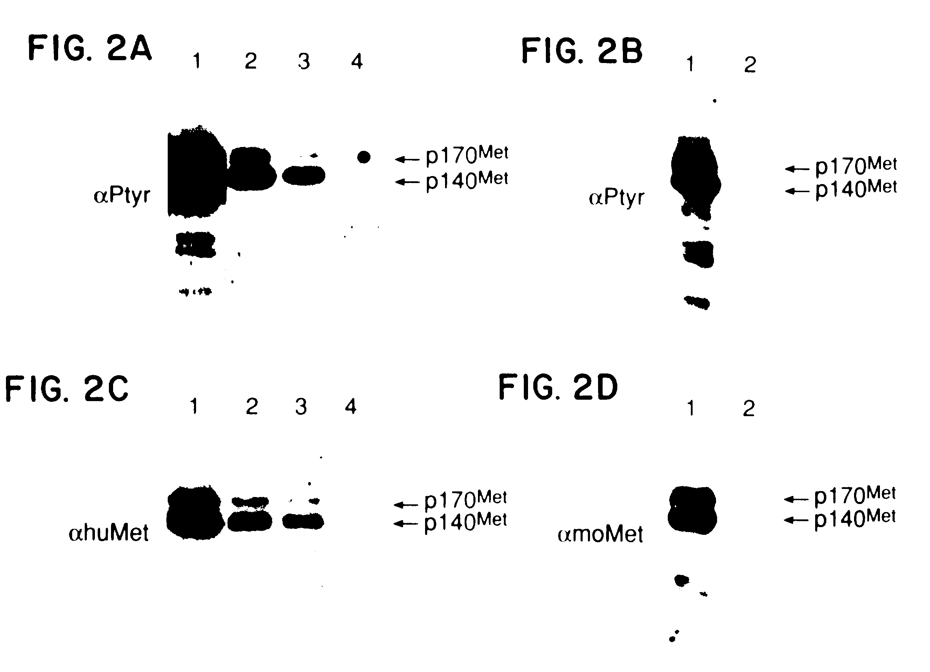

Figure 2 shows *met* product reactivity with anti-P-Tyr antibody. Near-confluent cells on a 100-mm dish were washed twice with cold TBS and lysed in 1 ml of lysis buffer (25 mM Tris-HCl, pH 8.0, 100 mM NaCl, 50 mM NaF, 1% Triton X-100, 10 µg/ml aprotinin, 10 µg/ml leupeptin, 1.25 mM PMSF, 1 mM sodium orthovanadate). One milligram of protein was immunoprecipitated with anti-C28 peptide antibody for Met^{hu}, (Gonzatti-Haces, M. *et al. Proc. Nat'l. Acad. Sci. U.S.A.* 85: 21-25 (1988)) (panels A and C) or peptide antibody SP260 for Met^{mu} (Iyer, A., *et al., Cell Growth* & *Diff.* 1: 87-95 (1990)) (panels B and D). After dissolving in SDS buffer, samples were separated by SDS-PAGE on 7.5% gels, transferred to Immobilon-P (Millipore), and probed with anti-P-Tyr antibody, 4G10, Morrison, D.K., *et al., Cell* 58: 649-657 (1989) (panels A and B), 19S monoclonal antibody (panel C), or mouse *met* peptide antibody, SP260 (panel D). The Immobilon filter was then incubated with ¹²⁵I-protein A (ICN) and subjected to autoradiography. Lanes 1-3 of panels A and C have three different lines of *met*^{hu}-transfected cells; lane 4 has NIH/3T3 control cells. Lane 1 of panels B and D are cells transfected with *met*^{mu}*.* Lane 2 has NIH/3T3 control cells.

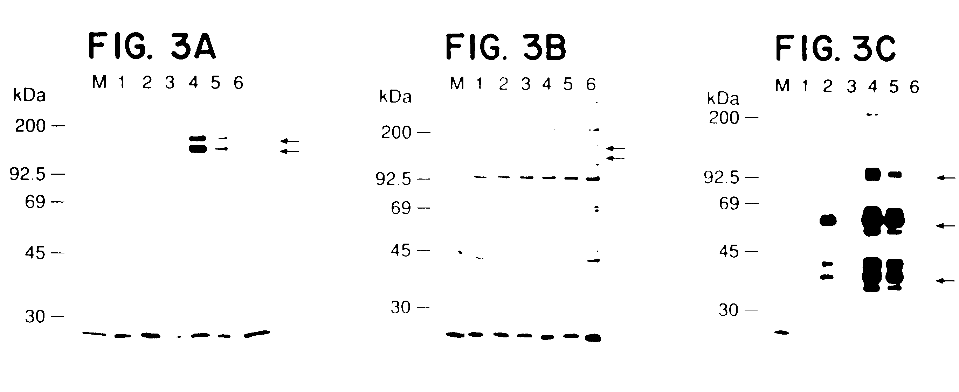

Figure 3 shows the characterization of Met^{hu} and HGF/SF^{hu} in NIH/3T3 tumor cells. Tumor cells were explanted and metabolically labeled with [³³S]methionine and [³⁵S]cysteine for 6 hours. Cell lysates were immunoprecipitated with either 19S monoclonal antibody (panel A), or peptide antibody SP260 (panel B). Then, 0.25 ml of the "6 hour" supernatants was concentrated threefold in Centricon (Amicon; 10K cut-off); the volumes were adjusted to 0.3 ml with RIPA buffer and the samples were immunoprecipitated with anti-HGF monoclonal antibody A3.1.2 (panel C). Lanes 1 and 3 are samples from two different lines of cotransfected cells before injection. Lane 2 is a tumor explant derived from the cells analyzed in lane 1; lanes 4 and 5 are tumor explants derived from the cells analyzed in lane 3. Lane 6 is a sample prepared from control NIH/3T3 cells. Arrows indicate the positions of p170^{met} and p140^{met} (panels A and B) and the positions of the 87 kDa (the precursor), 69 kDa, and 34 kDa HGF polypeptides (panel C).

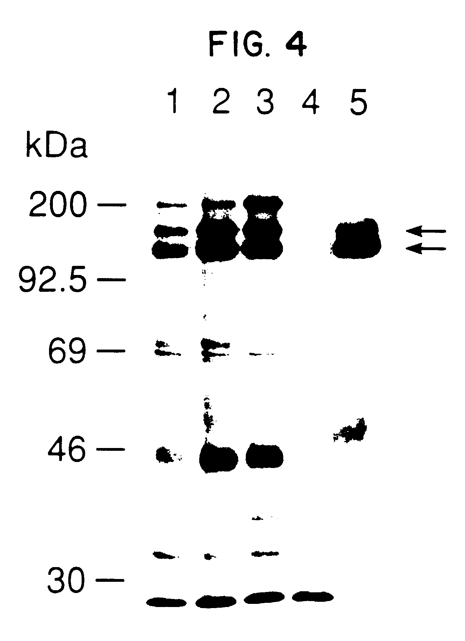

Figure 4 demonstrates the characterization of Met chimeric protein in NIH/3T3 cell tumors. Cells were metabolically labeled with [³⁵S]methionine and [³⁵S]cysteine for 6 hours and cell lysates were immunoprecipitated with 19S monoclonal antibody (lanes 1-4). In lane 1 are uninjected G418 (Gibco)-resistant cells transfected with mouse N-terminal/human C-terminal chimeric *met;* in lanes 2 and 3 are tumors derived from cells analyzed in lane 1; in lane 4, NIH/3T3 control cells; in lane 5, Western blot of the same cells as in lane 1, analyzed with anti-P-Tyr antibody (4G10) following immunoprecipitation with human anti-C28 peptide antibody.

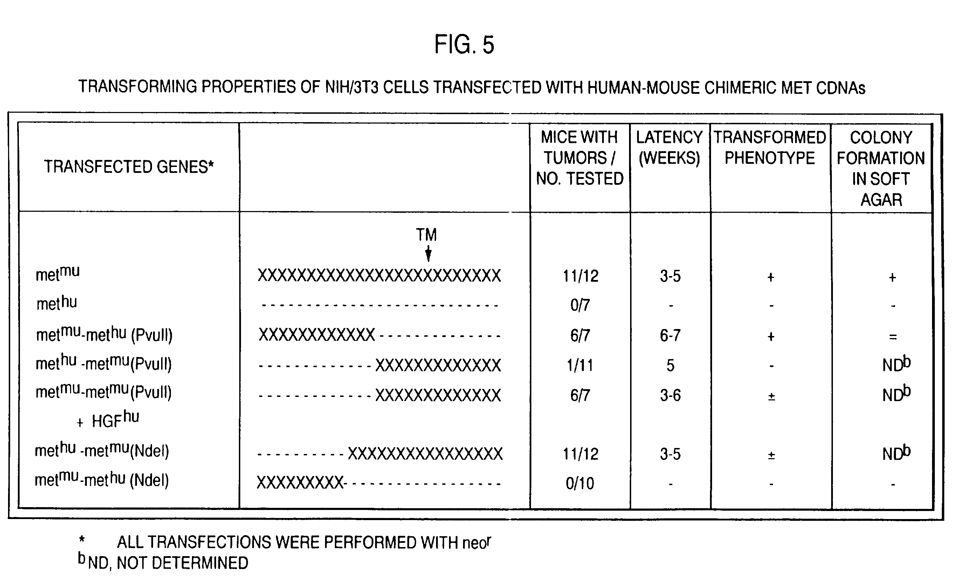

Figure 5 shows that the extracellular domain of Met^{mu} confers transforming potential onto Met^{hu} (line 3 compared to line 2) and the Met^{hu} extracellular domain is only transforming when co-transfected with HGF^{hu} (compare lines 4 to 5). Furthermore, the last two lines delineate the region of *met*^{mu} between the NdI-Pvu II sites as a region conferring transforming potential onto Met^{hu} (compare line 6 to line 4).

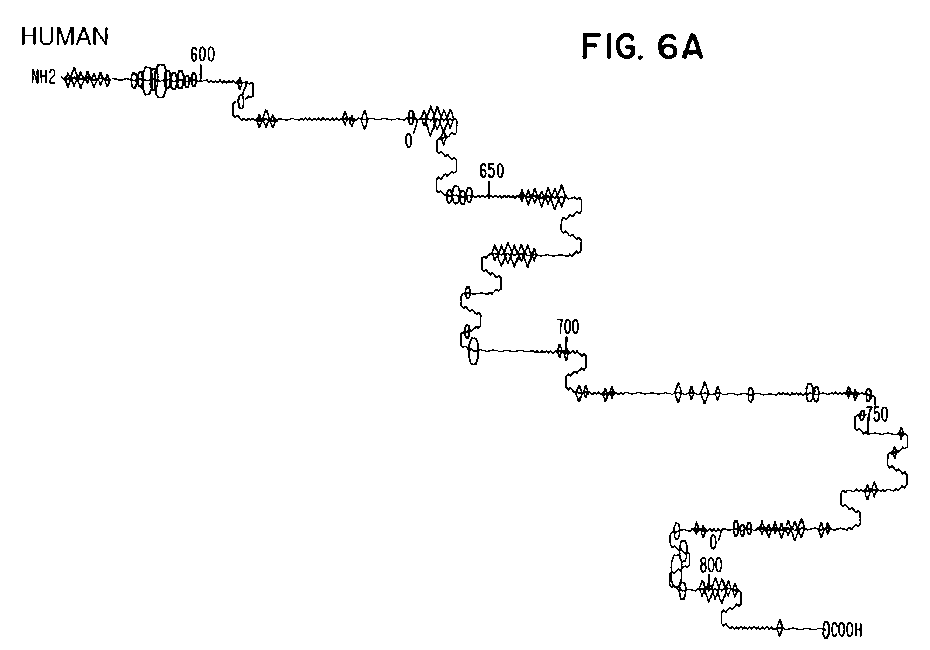

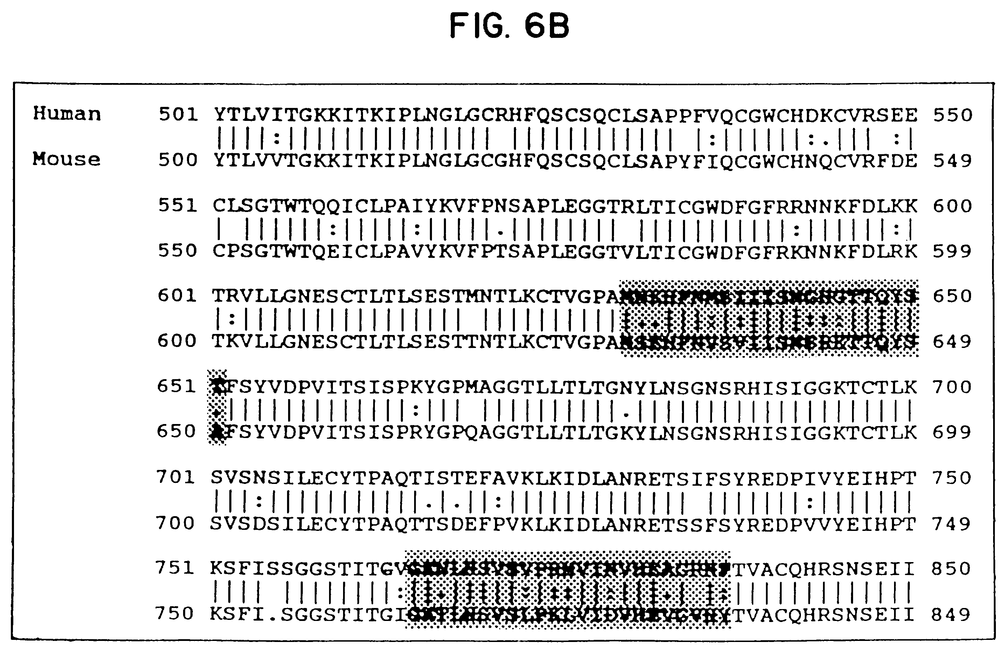

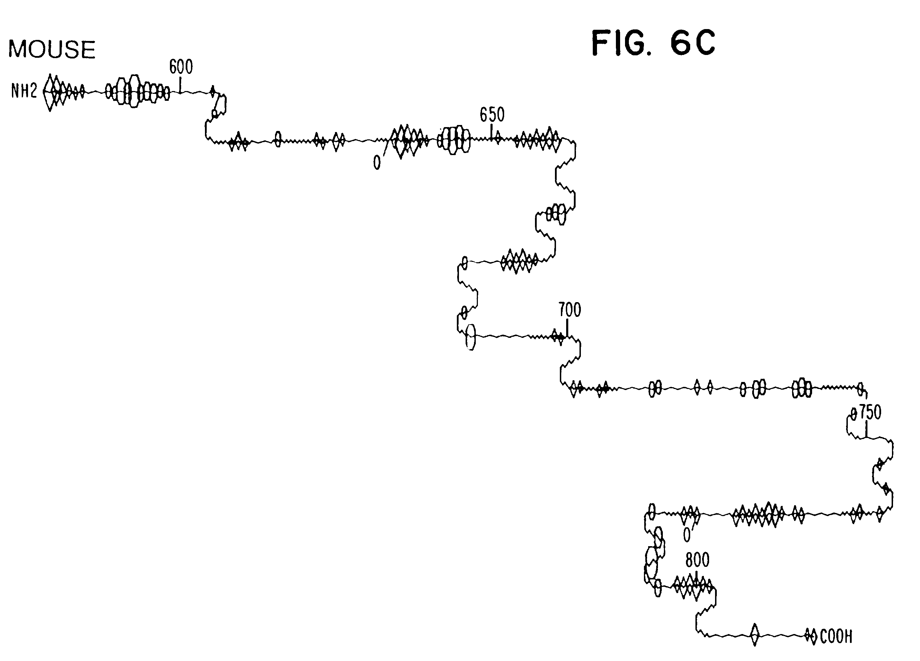

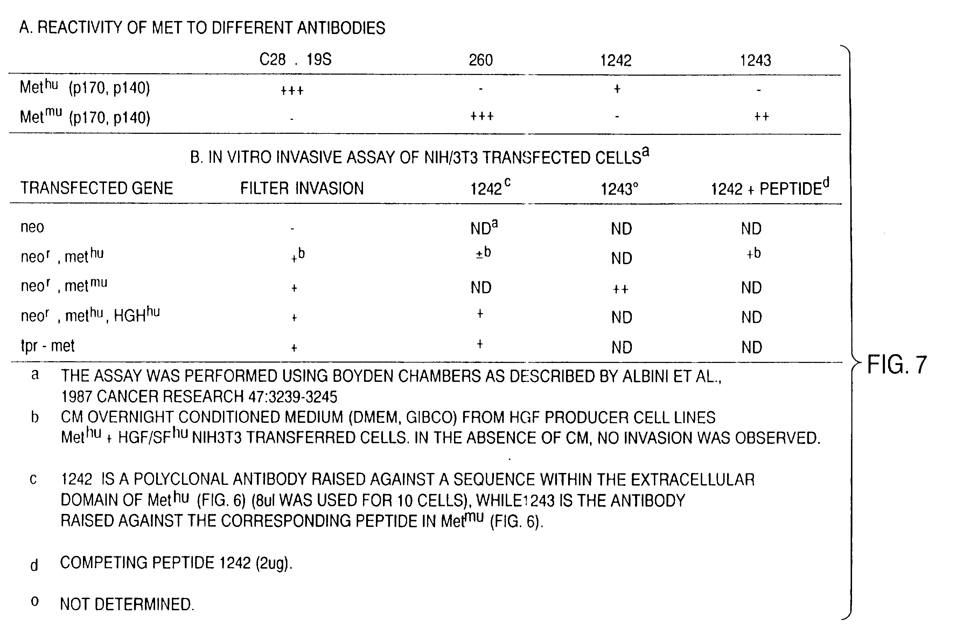

Figure 6 shows a comparison of the computer-generated predicted structure of the amino acid sequence between the conserved Nde I - Pvu II sites in the human Met (top) with mouse Met sequence (bottom). The amino acid sequence of human and mouse Met (between the Nde I - Pvu II sites) is depicted in the inset box - highlighted are regions where the amino acid sequence is less conserved (conservation is depicted by dashed lines between the human and mouse sequence). The less conserved domains within the Nde I - Pvu II segment of cDNA conferring transforming potential onto Met either reflect domains directly involved in ligand binding, or through structural characteristics modulate either ligand binding or activation of the receptor following ligand binding. Therefore, applicants generated polyclonal antibodies to synthetic peptides corresponding to these sequences. Antibodies to the first domain were generated in rabbits against human sequence (α 1240) and mouse sequence (α 1241); antibodies to the second domain were generated against human sequence (α 1242) and mouse sequence (α 1243). Only 1242 and 1243 precipitated human and murine *c-met* protein, respectively.

Figure 7 depicts the reactivity of human versus mouse Met to the human (C28, 19S, 1242) and mouse (260, 1243) specific antibodies (part A). Part B demonstrates that when mouse *met* cDNA is transfected into NIH/3T3 cells (which express HGF/SF^{mu}, endogenously) the cells are invasive in a Boyden chamber assay. (Albini et al. *Cancer Res.* 47: 3239-3245 (1987)). That is, the cells move across a filter coated with matrigel (a basement membrane-like compound). Cells transfected with the oncogenic form of *met* (tpr-*met*) are also highly invasive in this assay. For cells to invade across the filter, they must not only be motile, but must be able to degrade basement membrane components (and thus secrete enzymes such as collagenase or plasminogenase). *Met*^{hu} transfectants (line 2) are not invasive in the absence of human ligand, HGF/SF^{hu}, but are invasive when conditioned media from the HGF producer cell line is added. On the other hand, contransfectants of *met*^{hu} and HGF/SF^{hu} (line 5) are invasive in this assay

The second column on this figure shows that 1242 antibody to human Met within the domain described above inhibits filter invasion of the *met*^{hu} transfectants, co-cultured with conditioned media containing HGF/SF^{hu}. The last column shows that this inhibition is blocked in the presence of competing peptide. On the other hand, the invasive capacity of cotransfectants producing Met^{hu} and HGF^{hu} (line 4) is not blocked by the 1242 antibody, suggesting internal, intracellular autocrine activation of receptor by co-produced HGF/SF ligand.

Lastly, this figure shows that 1243 antibody did not block invasion of the filter by *met*^{mu} transfectants (line 3, column 3).

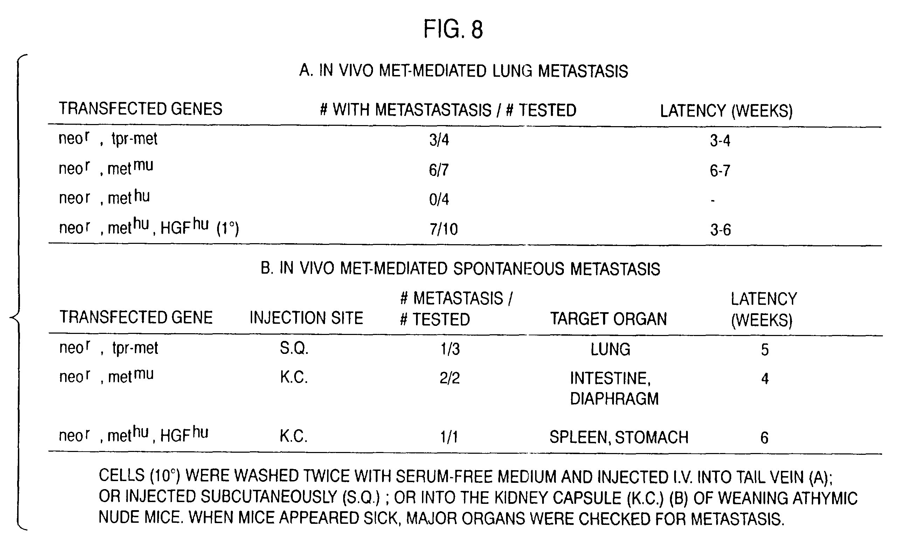

Figure 8 shows *in vivo* metastasis data showing that just as *met*^{mu} and tpr-met transfectants are tumorigenic *in vivo* (Figure 5), these cells are also metastatic in several types of assays (panels A and B). Furthermore, Met^{hu} (panel A) is not metastatic *in vivo* unless cotransfected with HGF^{hu} cDNA.

Figure 9 is a diagram depicting the construction of plasmids pRS2 and pRS24 which are co-transfected into NIH/3T3 cells in the production of HGF/SF, as described in Example 2.

### Detailed Description of the Preferred Embodiments

It is generally accepted in the art that for tumor cells to become "metastatic", a number of coordinated events must take place. For instance, metastasis involves the process whereby cancer cells penetrate the walls of the vascular and lymphatic circulatory systems, enter the circulatory system, lodge ("adhere") in a "downstream" capillary bed or lymph node and leave ("movement/mobility") the circulatory system to penetrate into normal stromal tissue. For these processes to occur, the tumor cell must acquire invasive properties. Invasive properties include (1) the ability of cells to adhere to the extracellular matrix (due to receptors on cell surface - such as the integrins); (2) the induction of destruction enzymes ("metalloproteases") which break through the extracellular matrix - such as collagenases and plasminogenases; and (3) the migration of cells through the "holes" created by enzymatic destruction. Increases in the levels of metalloproteases secreted by cells, increases in motogenicity of cells, and changes in tne pattern of expression of receptors, involved in attachment (integrins) have all been noted, and in some cases, correlate with the metastatic potential of human carcinoma cells. (L.A. Liotta, *Scientific American,* 54-63 (Feb. 1992)).

It is known that HGF/SF increases the levels of activity of at least two of these events: the secretion of collagenases and plasminogenases and motogenicity. Furthermore, HGF/SF is known to be angiogenic and angiogenesis is important for the survival of tumor cells. (J. Behrens *et al., J. Cell Biol.* 108: 2435-2447 (1989); K.M. Weidner *et al., J. Cell Biol.* 111: 2097-2108 (1990)). By the term "motogenesis" is meant the process whereby continuous sheets of cells disaggregate, change morphology and become motile. (E. Gherardi and M. Stoker, *Cancer Cells* 3: 227-232 (1991)). This phenomenon is also referred to as "scattering."

Applicants have demonstrated that HGF/SF induces motogenicity in two ways. *See* Example 1, below. First, as shown in Table 1, cells which display a classical scattering response to HGF/SF showed rapid phosphorylation of endogenous *met* receptor on tyrosine, suggesting that scattering is activated through the met receptor.

**Table 1.**

| Cell Line | Mitogenicity Index | Scattering (w/HGF) |
|---|---|---|
| Bix2NMA | 1 | +++ |
| Calu-1 | 1 | ± |
| SW620 | 1.7 | ++ |
| HT29 | 1 | + |
| HCT116 | 1.5 | + |
| ¹Mitogenicity index = ³H-thymidine incorporation in presence of HGF/³H-thymidine incorporation without HGF. | | |

Secondly, a vigorous testing was performed on mouse breast tumor "epithelial" cell line (C127 cells) transfected with mouse and human *met* cDNA's encoding the *met* proto-oncogene product, to show scattering in response to HGF/SF only in cells that express the *met* product from exogenously introduced *met* cDNA, but not in the non-transfected cells. These results are shown in Table 2:

**Table 2.**

| Cell Line | Mitogenicity Index | Scattering (w/HGF) |
|---|---|---|
| C127 | 3^{a} | -- |
| C127 - *met*^{hu} | 3 | ++ |
| C127 - *met*^{mu} | 3 | + |

| | | |
|---|---|---|
| ^{a}Stimulation of ³H-thymidine incorporation is presumed to be due to low levels of exogenous *c-met* product in C127 cells. | | |

In an embodiment of the present invention, applicants present a method for producing human HGF/SF. Specifically, applicants have discovered that mouse fibroblast NIH/3T3 cells express surprisingly high levels of HGF/SF from a transfected long terminal repeat (LTR) vector recombinant construct containing human HGF cDNA. Applicants have discovered that the highest levels of HGF are detected in NIH/3T3 cells when an LTR human *c-met* proto-oncogene (cDNA vector) is co-transfected with the human LTR/HGF construct and cells are derived from secondary tumors. *See* Example 2, below. One advantage of this cell line is that the transformed cells can grow to high cell density. Accordingly, this cell line produces extremely high levels of HGF/SF, about lmg of HGF/SF per liter (1250 units per ml). In comparison, another cell line derived from human keratinocytes, ndk cells, produces approximately 10µg per liter per 48 hours. *See J.* C. Adams, *et al., Science* 98: 385-394 (1991); E.M. Rosen, *et al., BBRC* 168(3): 1082-1088 (1990). It is therefore unexpected to obtain the yield of lmg/liter of HGF/SF from NIH/3T3 cells co-transfected with the recombinant vector constructs of the present invention.

Thus, one embodiment of the present invention relates to a method of producing HGF/SF comprising the steps of:
(a) transfecting NIH/3T3 cells with DNA encoding HGF/SF^{hu} and Met^{hu};
(b) introducing cells transfected in accordance with step (a) into a mammal, thereby generating a primary tumor;
(c) explanting and propagating cells of the primary tumor *in vitro;*
(d) selecting those cells propagated in accordance with step (c) which express high levels of HGF/SF^{hu} and high levels of Met^{hu};
(e) introducing cells selected in accordance with step (d) into a mammal thereby producing a secondary tumor;
(f) explanting and propagating cells of the secondary tumor *in vitro;* and
(g) obtaining HGF/SF^{hu} produced by the cells of step (f).

The DNA of the present invention comprises a first DNA vector comprising, in operable linkage, a promoter derived from a long terminal repeat of a retrovirus, DNA encoding the entire coding domain of human Met and a polyadenylation signal and a second DNA vector comprising, in operable linkage, a promoter derived from a long terminal repeat of a retrovirus, DNA encoding human HGF/SF and a polyadenylation signal.

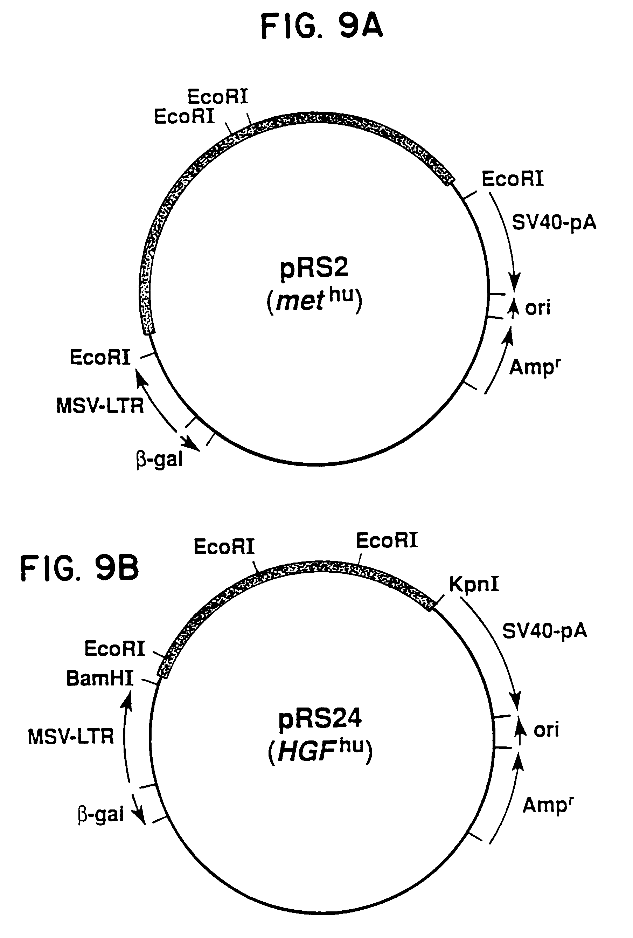

Although the production of the DNA vectors of the present invention may be accomplished by a variety of methods known to the skilled artisan, production is exemplified in Example 2, in the discussion of cDNA plasmid constructs and cell lines. The preferred DNA vectors of the present invention are depicted in Figure 9.

Cells transfected with HGF/SF^{hu} and Met^{hu} LTR-cDNA are introduced into mammals, according to methods well-known in the art, preferably by injection. *See* Blair, D.G., *et al., Science* 218: 1122-1125 (1982). The preferred mammals of the present invention are nude mice. Primary tumors which develop in these mammals after 5-10 weeks are explanted and propagated in *in vitro* culture, as described in Example 2, with regard to "nude mouse assays" *In vitro* propagated primary tumor cells are then subjected to immunoprecipitation analysis to ascertain which cells expressed high levels of HGF/SF^{hu} and high levels of Met^{hu}. *See* Figure 3 and the above description thereof. For instance, explanted cells may be metabolically labeled and then immunopreciptated with Met^{hu} monoclonal antibodies (monoclonal 19S), concentrated, then immunopreciptated with HGF/SF^{hu} monoclonal antibody A3.1.2. High levels of HGF/SF^{hu} and Met^{hu} are determined with reference to expression levels in the starting material. Thus, any level of expression higher than that observed in the starting material is considered "high" for purposes of this method. Cells expressing high levels of HGF/SF^{hu} and Met^{hu} are then introduced into mammals and secondary tumors which develop are explanted and propagated *in vitro,* according to methods well-known in the art. *See* Blair, D.G., *et al., Science* 218: 1122-1125 (1982).

HGF/SF^{hu} expressed by the explanted secondary tumor cells are then purified as described in Weidner *et al., J. of Cell Biol.* 111: 2097-2108 (1990), although other methods applicable to the present invention are well-known to the skilled artisan.

In yet another embodiment, the present invention relates to three C-terminal isoforms of mouse and human Met derived from NIH/3T3 cells transfected with *met* cDNA. Although these isoforms most likely resulted from post-translational processing, applicants cannot exclude possible rearrangements of the transfected cDNA. Several *met* products truncated in the C-terminal have been reported (Prat, M., *et al. Mol. Cell. Biol.* 11: 5954-5962 (1991)) but these products are different from the isoforms according to the present invention, because they do not react with C-terminal antibodies. Moreover, these isoforms are detected only in cells expressing high levels of *met,* indicating that they are present in low abundance.

### Example 1. The c-met proto-oncogene is the receptor associated with motogenicity.

The ability of HGF/SF to induce mitogenesis and/or scattering (motogenesis) in a number of human carcinoma cell lines that express *c-met* receptor was examined. Cell lines shown in Table 1, above, were plated in 96 well tissue culture plates at 1 X 10⁴- 5 X 10⁴ cells/ml and allowed to grow to near confluency. Wells were washed free of serum and cells starved for two days in serum-free media. The following day, cells were treated with either no sera, sera, or 10ng/ml HGF/SF for 16-18 hours, and then 1µCi of ³H-thymidine was added per well (5µCi/ml) for another six hours. The assay was terminated by washing wells free of label with ice-cold PBS, fixing the cells in 5% TCA, and solubilizing the DNA with 0.25 M NaOH. Samples were then counted in scintillation vials. The mitogenicity index is the ratio between thymidine incorporated in the absence of sera versus that stimulated by HGF. For motogenicity assays, cells were plated at 2 X 10³/ml in 16-well chamber slides (LabTek) and grown until about 60% confluent. Cells were than washed free of serum and incubated overnight in serum-free media +/- HGF/SF (10ng/ml). Slides were fixed with ice cold acetone for 10 minutes, stained with crystal violet (5 minutes), and rinsed well with water. Qualitative changes in "scattered phenotype" were recorded.

To demonstrate that the introduction of *c-met* into an epithelial cell line confers "scattering" activity onto the cells, C127 cells were transfected by lipofection (BRL) with either human *met* cDNA or murine *met* cDNA. Mitogenicity and/or motogenicity was measured as in Table 1. The results of this study are set forth in Table 2, above.

### Example 2. Production of HGF/SF in NIH/3T3 Cell Line

**cDNA plasmid constructs and cell lines.** *Met* cDNA plasmids were constructed in pMB1, a derivative (without the polylinker sequences) of pMEX, Oskam, R., *et al. Proc. Nat'l. Acad. Sci., USA* 85: 2964-2968 (1988); that contains the long terminal repeat (LTR) promoter from Moloney murine sarcoma virus (MSV) and the polyadenylation signal of simian virus 40. The *met*^{hu} plasmid was constructed by replacing an internal 300-bp *Eco*RI fragment with the 250-bp *Eco*RI fragment of pOK in the 4.6Kb *met*^{hu} sequence containing the open reading frame. Park, M., *et al. Proc. Nat'l. Acad. Sci.* USA 84: 6379-6383 (1987); Rodriguez *et al., Mol. Cell Biol.* 11: 2962-2970 (1991). The *met*^{mu} plasmid contains the entire 4.6-Kb mouse *met* open reading frame. Iyer, A., *et al. Cell Growth & Diff.* 1:87-95 (1990). Chimeric human/mouse *met* constructs were made using the conserved *Pvu*II site (amino acid 807). A *HGF*^{hu} plasmid was constructed by inserting the 2.3 kb *Bam*H1-*Kpn*I fragments of the human *HGF* sequence into the *Bam*H1-*Kpn*I sites of pMEX. Nakamura, T., *et al. Nature* 342: 440-443 (1989); Oskam, R., *et al. Proc. Nat'l. Acad. Sci., USA* 85: 2964-2968 (1988). NIH/3T3 490 cells were grown in DMEM (Gibco) with 8% calf serum (Gibco).

**DNA transfection.** The calcium phosphate method of DNA transfection (Cooper, C.S., *et al. Nature* 311:29-33 (1984)) was carried out by mixing plasmid DNA (2 µg in 75 µl of water containing 8 µg of calf thymus carrier DNA) with 75 µl of 0.67 M CaCl₂. This mixture was added dropwise to 0.15 ml of solution H (0.27 M NaCl, 0.01 M KCl, 0.0014 M Na₂HPO₄.7H₂O, 0.012 M dextrose) with continuous agitation. After remaining at room temperature for 30 minutes, the mixture was added to cells having about 70% confluence on a 35-mm dish containing 1 ml medium with 0.01 M Hepes buffer. Cells were incubated at 37°C for 4 hours, then treated with 15% glycerol (v/v) in solution H for 2 minutes. For G418 selection, cells were re-fed with DMEM and 8% calf serum overnight and subsequently transferred to three 60mm dishes. After 24 hours incubation, cells were fed with medium containing 400 µg/ml G418 (Gibco) twice a week.

**Northern analysis.** RNA was isolated using RNAsol (CINNA/BIOTECX). Twenty micrograms of RNA was subjected to electrophoresis on 1% denaturing formaldehyde agarose gels followed by transfer to nitrocellulose filters (Schleicher and Schuell). Blots were hybridized at 42°C for 15 hours to ³²P-labeled randomly primed DNA probes (10⁹ cpm/µg) in 30% formamide, 6x saline sodium citrate (SSC), 5x Denhardt's solution, 50mM sodium phosphate (pH 6.8), and sonicated salmon sperm DNA (250 µg/ml). After hybridization, filters were washed twice in lx SSC and 0.1% SDS at room temperature and in lx SSC and 0.1% SDS at 50°C. Filters were dried and exposed to X-ray films for 1-3 days at -70°C.

**Immunoprecipitation.** Near-confluent cells were labeled with 0.25 mCi of Translabel (ICN) (1 ml/35-mm dish) for 4 to 6 hours in DMEM lacking methionine and cysteine (Gibco). The labeled cells were lysed in 0.5 ml of RIPA buffer [1% Triton X-100, 1% sodium deoxycholate, 0.1% sodium dodecylsulfate (SDS), 0.15 M NaCl, 0.02 M NaPO₄, pH 7.2, 1 mM phenylmethylsulfonyl fluoride (PMSF), 2 mM EDTA, 50 mM NaF, 30 mM sodium pyrophosphate]. Clarified lysates having equal radioactive counts were immunoprecipitated with 19S monoclonal anti-met antibody, Faletto, D.L. *et al. Oncogene* 7: 1149-1157 (1991) at 4°C overnight. Immunoprecipitates were complexed to protein G-sepharose (Gibco), then washed twice with RIPA buffer and high-salt buffer (1 M NaCl, 10 mM Tris-HCl, pH 7.2, 0.5% Triton). The immunocomplexes were solubilized by boiling in SDS sample buffer in the presence of 5% β-mercaptoethanol. Samples were analyzed by SDS-polyacrylamide gel electrophoresis followed by treatment with fluorographic enhancer (Amplify^{TM}, Amersham) and fluorography with an intensifying screen at -70°C.

SP260 is a peptide antibody made from rabbit antisera, directed against the C-terminal 21 amino acids of *met*^{mu}*.* (Iyer, A., *et al. Cell Growth & Diff.* 1: 87-95 (1990)). A3.1.2 is an anti-human recombinant HGF monoclonal antibody (IgG, subclass G2a).

**Pulse chase analysis.** Near-confluent cells were labeled for 45 min with 0.25 mCi of Translabel in 1 ml (per 35-mm dish) DMEM lacking methionine and cysteine. The cells were washed twice and chased with complete medium for 3 hours, then lysed and subjected to immunoprecipitation analysis.

**Surface iodination.** Near-confluent cells were labeled with Na ¹²⁵I in the presence of Iodo-Gen (Pierce). Twenty microliters of the Iodo-Gen reagent (10 mg/ml in chloroform) was added to the bottom of a I-dram vial and dried with a stream of nitrogen. The Iodo-Gen was then dissolved in 1 M Tris with 10 mM EDTA (pH 7.5) and added to the cell pellet. Na ¹²⁵I (0.5 mCi) was added to the reaction for 10 minutes. The labeled cells were washed three times with PBS, then lysed with RIPA buffer and subjected to immunoprecipitation analysis.

**Western analysis**. Near-confluent cells on a 100-mm dish were washed twice with cold TBS (10 mM Tris pH 8.0, 150 mM NaCl) and lysed in 1 ml of lysis buffer (25 mM Tris-HCl, pH 8.0, 100 mM NaCl, 50^{·}mM NaF, 1% Triton X-100, 10 µg/ml aprotinin, 10 µg/ml leupeptin, 1.25 mM PMSF, 1 mM vanadate). One milligram of protein was immunoprecipitated with anti-C28 anti-Met^{hu} polyclonal antibody (Gonzatti-Haces, M. *et al., Proc. Nat'l. Acad. Sci. U.S.A.,* 85: 21-25 (1988)) and subjected to Western analysis with either anti-phosphotyrosine (anti-P-Tyr) antibody, 4G10 (Morrison, D.K., *et al., Cell,* 58: 649-657(1989)), or human or mouse met-specific antibodies, 19S monoclonal (Faletto, D.L., *et al.,* Oncogene 7: 1149-1157 (1991)) or SP260 (Iyer, A., *et al.,* Cell *Growth & Diff. 1:* 87-95 (1990)), respectively. ¹²⁵I-protein A (Amersham) was used to detect positive bands according to the manufacturer's instructions.

**Nude mouse tumor assay**. The assay was performed as previously described (Blair, D.G., *et al., Science* 218: 1122-1125 (1982)). Transfected and G418 selected NIH/3T3 cells (10⁶) were washed twice and resuspended in 0.1 ml of serum-free medium. The cells were injected onto the back of weanling athymic nude mice (Harlan Sprague Dawley, Inc.). Tumor formation was monitored each week for up to 10 weeks. Tumors were explanted when they reached 15 mm in size, and the tumor cells were subjected to immunoprecipitation analysis.

**Soft-agar assay.** Soft-agar growth assay was carried out by modification of Blair *et al.*, *Virology* 95: 303-316 (1979). Briefly, trypsinized cells were suspended at 2 x 10⁵ and 2 x 10⁴ cells in 8 ml of DMEM (Gibco) with 10% calf serum and 0.24% purified agar (DIFCO) and quickly transferred to a duplicate 60-mm dish containing a hardened base layer of DMEM with 10% calf serum and 0.27% agar. Plates were fed with 2 ml of DMEM with 10% calf serum and 0.27% agar at weekly intervals. Colonies were counted microscopically after 3 weeks incubation at 37°C.

**Expression of Met**^{**hu**} **and Met**^{**mu**} **in NIH/3T3 cells.** Plasmids containing *met*^{hu} protooncogene cDNA were cotransfected with pSV2neo into NIH/3T3 cells and G418 - resistant cells were screened for human or mouse Met expression by immuno-precipitation analyses. These analyses show that both human and mouse p170^{met} and p140^{met} are expressed in transfected NIH/3T3 cells (Fig. 1A, lanes 2 and 3, respectively). Similar levels of *met*^{*mu*} expression in NIH/3T3 cells has been reported (Iyer, A., *et al., Cell Growth & Diff.* 1:87-95 (1990)). Applicants found little or no expression of the endogenous Met^{mu} in the G418-resistant cells expressing Met^{hu}. Appropriate processing of Met^{hu} and Met^{mu} was examined by pulse-chase labeling experiments and these studies showed that p170^{met} synthesized during a 45 minute pulse (Fig. 1B, lanes 1 and 3) was efficiently processed after 3 hours into the mature p140^{met} (lanes 2 and 4). Moreover, human and mouse Met were localized on the cell surface. Applicants labeled intact cells with Na¹²⁵I and immunoprecipitation of the lysates showed that both forms, p140^{met} and p170^{met} were iodinated (Fig. 1C). Thus, human or mouse Met expressed in NIH/3T3 cells is correctly processed and localized on the cell surface. These analyses also show that pl70^{met} arrives at the cell surface uncleaved. The iodination of p170^{met} did not result from lysed cells, since under similar iodination conditions the cytoplasmic *tpr-met* oncoprotein was not detected. (Gonzatti-Haces, M., *et al., Proc. Nat'l. Acad. Sci. U.S.A.* 85: 21-25 (1988)). Furthermore, p170^{met} expressed in Okajima cells, a human gastric carcinoma cell line that overexpresses *met*^{hu} is also labeled by surface iodination (data not shown), but the ratio of p170^{met} to p140^{met} labeled was less when compared with NIH/3T3 cells.

**Constitutive tyrosine phosphorylation of *met* in NIH/3T3 cells**. By Northern hybridization analyses, applicants detected *HGF/SF* mRNA expression in NIH/3T3 cells and in cells transfected with either *met*^{hu} or *met*^{mu}. Applicants observed the same level of HGF/SF mRNA expression, suggesting that overexpression of *met*^{hu} or *met*^{mu} in the G418 selected lines did not affect endogenous *HGF/SF* expression. These results also suggested that Met might be activated in an autocrine fashion. Therefore, applicants examined whether the Met^{hu} and Met^{mu} expressed in these cells reacted with anti-P-Tyr antibody. Extracts from cell lines expressing Met^{hu} and Met^{mu} were subjected to immunoprecipitation with human- or mouse-specific peptide antibodies followed by Western analyses using either anti-P-Tyr antibody (Fig. 2A and B), or human (Fig. 2C), or mouse-specific (Fig. 2D) Met antibody. These analyses show that both p170^{met} and p140^{met} of Met^{hu} and Met^{hu} reacted strongly with anti-P-Tyr antibody. This is the first example demonstrating tyrosine phosphorylation of p170^{met} react with anti-P-Tyr antibody. One line expressed Met^{hu} at very high levels (Fig. 2A and C, lane 1). This was an exception since all other Met^{hu} lines expressed levels comparable to lines analyzed in Fig. 2A and C, lanes 2 and 3. Additional C-terminal Met protein products (p85, p75, and p65) were detected with C-terminal antibodies in cells expressing either mouse or human *met* (Fig. 2A-D, lane 1).

**Tumorigenicity of *met* in NIH/3T3 cells.** Applicants observed that NIH/3T3 cell cultures expressing Met^{mu}, but not Met^{hu}, were transformed (Iyer, A., et *al., Cell Growth & Diff.* 1: 87-95 (1990)) as shown in Figure 5. This was confirmed by testing for their tumorgenicity in nude mice following transfection and G418 selection. NIH/3T3 cells expressing met^{mu} were highly tumorigenic as shown in Table 3, below, while cells expressing *met*^{hu} were low in tumorigenicity and only in one line out of eight tested produced tumors.

**Table 3.**

| Tumorigenicity of NIH/3T3 Cells Transfected With *met*^{hu} or *met*^{mu} cDNA | | | |
|---|---|---|---|
| Cells* | Transfected genes | Mice with tumors/No. tested | Latency (weeks) |
| NIH/3T3 | neo^{r} | 0/8 | |
| 123-1 | neo^{r}, *met*^{*hu*} | 2/23 | 5 |
| 121-5 | neo^{r}, *met*^{mu} | 12/12 | 3-5 |

| | | | |
|---|---|---|---|
| Cells (10⁶) were washed twice with serum-free medium and injected subcutaneously on the back of weanling athymic nude mice. Tumor formation was monitored each week up to 10 weeks. | | | |

These tumorigenic lines, before injection, produced high levels of Met^{hu} (Fig. 2A and C, lane 1) compared to lines that were not tumorigenic (Fig. 2A and C, lanes 2 and 3). The tumor explants of this line displayed even higher levels of Met^{hu}. The levels of a Met^{mu} line before injection is shown for comparison (Fig. 2B and D, lane 1). Cell lines expressing lower levels of Met^{mu} are also tumorigenic in this assay. The two tumors generated by line 123-1 (Table 3) showed increased levels of endogenous Met^{mu} expression and reduced levels of Met^{hu}, suggesting that they may have arisen through mouse *met* amplification as previously described. Cooper, C.S., *et al., EMBO J.* 5: 2623-2628 (1986); Heldin, C.-H. *et al., Eur. J. Biochem.* 184: 487-496 (1989). Applicants conclude that Met^{hu} is poorly tumorigenic in NIH/3T3 cells.

**Tumorigenicity of NIH/3T3 cells cotransfected with** *met*^{hu} and *HGF/SF*^{hu}*.* One explanation for the low tumorigenicity of *met*^{hu}*-* transfected NIH/3T3 cells is that *met*^{hu} receptor activation by endogenous HGF/SF^{mu} may not provide sufficient signal. Applicants therefore tested whether transfection of both *met*^{hu} and *HGF/SF*^{hu} cDNAS would increase tumorigenicity through an autocrine mechanism. These analyses show that NIH/3T3 cells cotransfected with *met*^{hu} and *HGF/SF*^{hu} are highly tumorigenic (Table 4).

**Table 4.**

| Tumorgenicity of NIH/3T3 Cells Transfected With *met*^{hu} ± *HGF/SF*^{hu} cDNAs | | | |
|---|---|---|---|
| Cells | Transfected genes | Mice with tumors/No. tested | Latency (weeks) |
| NIH/3T3 | neo^{r} | 0/6 | - |
| 132-4 | neo^{r}, *met*^{hu} | 0/6 | - |
| 132-3, 137-5 | neo^{r}, *met*^{hu}, HGF^{hu} | 17/19 | 4-6 |
| 137-4 | neo^{r}, HGF^{hu} | 3/7 | 7 |

Moreover, the tumor cells showed increased levels of both Met^{hu} and HGF/SF^{hu} (Fig. 3A and C, respectively; lanes 2, 4, and 5). In the tumor explants characterized in lanes 4 and 5, high levels of both Met^{hu} and HGF/SF^{hu} are expressed. None of the cell lines showed amplification of endogenous *met*^{mu} (Fig. 3B). Applicants found that *HGF*/*SF*^{hu}-transfected NIH/3T3 cells also produced several tumors, but the levels of *HGF/SF*^{hu} product expressed in tumor cells derived from explants was not as high as the levels expressed in tumors from the *met*^{hu}*-HGF/SF*^{hu} cotransfection experiments (Fig. 3C). In one out of five HGF/SF^{hu} tumors, elevated levels of endogenous *met*^{hu} was detected.

**Tumorigenicity of chimeric human/mouse *met* in NIH/3T3 cells.** To test whether the ligand binding domain influenced tumorigenicity, applicants generated chimeric human/mouse *met* receptor molecules and tested their tumorigenicity in nude mice (Figure 5). Applicants used a conserved PvuII site in the external domain adjacent to the transmembrane coding sequences to make these recombinants. It was found that when the mouse external ligand-binding domain was linked to the human transmembrane and tyrosine kinase domains, the chimeric receptor displayed tumorigenic activity equivalent to that of the Met^{mu} (Figure 5). Explants of these tumors showed an increased level of chimeric Met protein that was recognized with human antibody directed against the human tyrosine kinase domain (Fig. 4, lanes 2 and 3). No evidence for *met*^{mu} amplification was observed in these tumors, and the chimeric product was recognized by Western analysis with anti-P-Tyr antibody (Fig. 4, lane 5).

In contrast to the high tumorigenicity of the chimera with the mouse Met external ligand-binding domain, the reciprocal human N-terminal/mouse C-terminal chimera was poorly tumorigenic. However, as with the Met^{hu}, when this chimera was cotransfected with *HGF/SF*^{hu} cDNA, efficient tumor formation was observed (Figure 5). Applicants also show that cells expressing the met^{mu}-met^{hu} chimera, before injecting into nude mice, are transformed and form colonies in soft agar like *met*^{mu}-transfected cells. The met^{hu}-met^{mu} cells do not display a transformed phenotype unless coexpressed with HGF/SF. Applicants concluded that the Met^{mu} external ligand-binding domain is a major factor in determining tumorigenicity.

While the foregoing invention has been described in some detail for purposes of clarity and understanding, it will be appreciated by one skilled in the art from a reading of this disclosure that various changes in form and detail can be made without departing from the true scope of the invention.

## Claims

1. A method of producing HGF (hepatocyte growth factor)/SF (scatter factor) comprising the steps of:
(a) transfecting NIH/3T3 cells with DNA encoding HGF/SF^{hu} and Met (met protooncogene protein)^{hu};
(b) introducing cells transfected in accordance with step (a) into a mammal, thereby generating a primary tumor;
(c) explanting and propagating cells of said primary tumor *in vitro;*
(d) selecting those cells propagated in accordance with step (c) which express high levels of HGF/SF^{hu} and high levels of Met^{hu};
(e) introducing cells selected in accordance with step (d) into a mammal, thereby producing a secondary tumor;
(f) explanting and propagating cells of said secondary tumor *in vitro;* and
(g) obtaining HGF/SF^{hu} produced by said cells of step (f).

2. A cell line co-transfected with a first DNA vector comprising, in operable linkage, a promoter derived from a long terminal repeat of a retrovirus, DNA encoding the entire coding domain of human Met (met protooncogene protein) and a polyadenylation signal, and a second DNA vector comprising, in operable linkage, a promoter derived from along terminal repeat of a retrovirus, DNA encoding human HGF (hepatocyte growth factor)/SF (scatter factor) and a polyadenylation signal.

3. The cell line of claim 2 which is the NIH/3T3 cell line.

## Patentansprüche

1. Methode zur Herstellung von HGF (Hepatozytwachstumsfaktor) / SF (Verbreitungsfaktor), umfassend die folgenden Stufen:
(a) Transfektion von NIH/3T3-Zellen mit DNA, das für HGF/SF^{hu} und Met^{hu} (met Protoonkogen-Protein) kodiert;
(b) Einführung von Zellen, die gemäss Stufe (a) transfiziert worden sind, in einen Säugetier, um einen primären Tumor zu bilden;
(c) Explantation und Propagation der Zellen des primären Tumors *in vitro;*
(d) Auswahl derjenigen Zellen, die gemäss Stufe (c) propagiert worden sind, die hohe Mengen von HGF/SF^{hu} und hohe Mengen von Met^{hu} ausdrücken;
(e) Einführung der Zellen, die gemäss Stufe (d) ausgewählt worden sind, in einen Säugetier, um einen sekundären Tumor zu bilden,
(f) Explantation und Propagation der Zellen des sekundären Tumors *in vitro;* und
(g) Isolierung des HGF/SF^{hu}, der von den Zellen gemäss Stufe (f) produziert worden ist.

2. Zelllinie, die mit einem ersten DNA-Vektor co-transfiziert worden ist, umfassend, in operabler Verbindung, einen Promotor, der von einer langen terminalen Wiederholung eines Retrovirus abgeleitet worden ist, DNA, das für die gesamte kodierende Domäne eines menschlichen Met (met Protoonkogen-Protein) kodiert, und ein Polyadenylierungssignal, und einem zweiten DNA-Vektor umfassend, in operabler Verbindung, einen Promotor, der von einer langen terminalen Wiederholung eines Retrovirus abgeleitet worden ist, DNA, das für humanen HGF (Hepatozytwachstumsfaktor) / SF (Verbreitungsfaktor) kodiert, und ein Polyadenylierungssignal.

3. Zelllinie gemäss Anspruch 2, die die NIH/3T3-Zelllinie ist.

## Revendications

1. Méthode pour la préparation du HGF (facteur de croissance d'hépatocytes) / SF (facteur de diffusion), comprenant les étapes suivantes:
(a) la transfection de cellules NIH/3T3 avec de l'ADN codant pour le HGF/SF^{hu} et le Met^{hu} (protéine met protooncogène);
(b) l'introduction dans un mammifère des cellules transfectées selon l'étape (a), ce qui produit une tumeur primaire;
(c) l'explantation et la propagation *in vitro* de cellules provenant de ladite tumeur;
(d) la sélection des cellules propagées selon l'étape (c) qui expriment des niveaux élevés du HGF/SF^{hu} et des niveaux élevés du Met^{hu};
(e) l'introduction dans un mammifère de cellules sélectionées selon l'étape (d), ce qui produit une tumeur secondaire;
(f) l'explantation et la propagation *in vitro* de cellules provenant de ladite tumeur secondaire; et
(g) l'obtention du HGF/SF^{hu} produit par les cellules provenant de l'étape (f).

2. Ligne cellulaire co-transfectée par un premier vecteur d'ADN comprenant, en liaison opérable, un promoteur dérivé d'une répétition longue terminale d'un rétrovirus, de l'ADN codant pour la totalité du domaine codant du Met (protéine met protooncogène) et un signal de polyadénylation, et un second vecteur d'ADN comprenant, en liaison opérable, un promoteur dérivé d'une répétition longue terminale d'un rétrovirus, de l'ADN codant pour le HGF (facteur de croissance d'hépatocytes) / SF (facteur de diffusion) humain, et un signal de polyadénylation.

3. Ligne cellulaire selon la revendication 2 qui est la ligne cellulaire NIH/3T3.
